# EUROPEAN PATENT APPLICATION

(11) **EP 4 603 952 A1**
(43) Date of publication of application: **20.08.2025**
(21) Application number: 23877092.9
(22) Date of filing: 20.09.2023
(51) Int. Cl.: G06F 3/01, A47J 44/00, A63B 69/00, A63H 11/00, B25J 9/22, B25J 11/00, B25J 13/06, G09B 19/00

(54) **AUTONOMOUS DRIVING 10 FINGER**

(30) Priority: 11.10.2022 JP 2022163597; 08.11.2022 JP 2022178992; 30.03.2023 JP 2023054737
(71) Applicant: SoftBank Group Corp., Tokyo 105-7537 (JP)
(72) Inventor: SON, Masayoshi, Tokyo 105-7537 (JP)
(74) Representative: Meissner Bolte Partnerschaft mbB
(86) International application number: PCT/JP2023/034035
(87) International publication number: WO 2024/080086

(57) **Abstract**

Provided is a control device including an actuating unit that moves an object; an information acquisition unit that acquires a plurality of pieces of information; and a control unit that controls the actuating unit by using the information acquired by the information acquisition unit and AI. The control unit may control the actuating unit in units of 1/1 billion seconds by using the information and AI, and the actuating unit may drive a vehicle. The actuating unit may be configured to be worn by a human, and the control unit may cause the actuating unit to assist a motion of the human who drives a vehicle. The control unit may control the actuating unit by using the information and AI, and cause the actuating unit to reproduce a dish, perform surgery, or perform a factory work.

## Description

### Cross-Reference to Related Applications

The present application is based on Japanese Patent Application No. 2022-163597 filed on October 11, 2022, Japanese Patent Application No. 2022-178992 filed on November 8, 2022, and Japanese Patent Application No. 2023-054737 filed on March 30, 2023, the contents of which are incorporated herein by reference.

### Technical Field

The present invention relates to an autonomous driving 10 finger, and a smart robot having ten fingers that implements an ultra-high performance information acquisition unit.

### Background Art

Patent Literature 1 describes a vehicle having an automatic driving function.

### Citation List

### Patent Literature

Patent Literature 1: JP 2022-035198 A

### Summary of Invention

According to one embodiment of the present invention, a control device is provided. The control device may include an actuating unit that moves an object. The control device may include an information acquisition unit that acquires a plurality of pieces of information. The control device may include a control unit that controls the actuating unit by using information acquired by the information acquisition unit and AI.

The control unit may control the actuating unit in units of 1/1 billion seconds by using the information and AI, and the actuating unit may drive a vehicle. The actuating unit may be configured to be worn by a human, and the control unit may cause the actuating unit to assist a motion of the human who drives a vehicle.

In any of the control devices, the information acquisition unit may acquire at least one of heat, a shape, a color, a texture, and an odor of a dish as information, and the control unit may control the actuating unit by using the information and AI and cause the actuating unit to reproduce a dish. The actuating unit may be configured to be worn by a human, and the control unit may cause the actuating unit to assist a motion of the human who cooks.

In any of the control devices, the information acquisition unit may acquire an operation of surgery of a doctor, and the control unit may control the actuating unit by using the information and AI and cause the actuating unit to perform surgery. The actuating unit may be configured to be worn by a human, and the control unit may cause the actuating unit to assist a motion of a human who performs surgery.

In any of the control devices, the information acquisition unit may acquire an operation of a factory worker, and the control unit may control the actuating unit by using the information and AI and cause the actuating unit to perform a factory work. The actuating unit may be configured to be worn by a human, and the control unit may cause the actuating unit to assist a motion of the human who performs a factory work.

According to one embodiment of the present invention, there is provided a program for causing a computer to function as the control device.

Note that the above summary of the invention does not enumerate all the necessary features of the present invention. Further, a sub-combination of these feature groups can also be an invention.

### Brief Description of Drawings

[Figure 1]Fig. 1 schematically shows an example of a functional configuration of a control device 100.
[Figure 2]Fig. 2 schematically shows an example of a network configuration according to the present embodiment.
[Figure 3]Fig. 3 schematically shows examples of sensors attached to a first joint and a second joint of a finger of a robot.
[Figure 4]Fig. 4 schematically shows an example of an AI robot controlled by the control device 100.
[Figure 5]Fig. 5 schematically shows ultra-high performance information acquisition according to the present embodiment.
[Figure 6]Fig. 6 schematically shows ultra-high performance automatic driving realized by the AI robot according to the present embodiment.
[Figure 7]Fig. 7 schematically shows perfect speed control realized by control by the control device 100 according to the present embodiment.
[Figure 8]Fig. 8 schematically shows perfect bell curves.
[Figure 9]Fig. 9 is a schematic view of perfect cruising.
[Figure 10]Fig. 10 is a schematic view of perfect cruising.
[Figure 11]Fig. 11 is a schematic view of perfect cruising.
[Figure 12]Fig. 12 is a schematic view of perfect cruising.
[Figure 13]Fig. 13 is a schematic view of perfect cruising.
[Figure 14]Fig. 14 is a schematic view of perfect cruising.
[Figure 15]Fig. 15 is a schematic view of perfect cruising.
[Figure 16]Fig. 16 schematically shows an example of a hardware configuration of a computer 1200 functioning as the control device 100 or a central brain SoC.
[Figure 17]Fig. 17 is a view showing a configuration of an operation reproducing device 1 according to a second embodiment.
[Figure 18]Fig. 18 is a view showing an information acquisition unit 20 of the operation reproducing device 1 according to the second embodiment.
[Figure 19]Fig. 19 is a view showing a robot 100 realized by the operation reproducing device 1 according to the second embodiment.
[Figure 20]Fig. 20 is a flowchart of an operation example of the operation reproducing device 1 according to the second embodiment.
[Figure 21]Fig. 21 is a view showing a configuration of an operation learning support device according to a third embodiment.
[Figure 22]Fig. 22 is a view showing a robot realized by the operation learning support device according to the third embodiment.
[Figure 23]Fig. 23 is a flowchart showing an operation example of the operation learning support device according to the third embodiment.
[Figure 24]Fig. 24 is a screen example showing a comparison result between a motion of a user and a motion of a chef according to the third embodiment.

### Description of Embodiments

Hereinafter, the present invention will be described through embodiments of the invention, but the following embodiments do not limit the invention according to the claims. In addition, not all combinations of features described in the embodiments are essential to the means for solving the invention.

### (1) First Embodiment

In the present embodiment, an AI robot realizes ultra-high performance automatic driving or takes the place of a first-class chef or a first-class doctor, based on information acquired by an ultra-high performance information acquisition unit. In the AI robot, the AI robot itself may operate, or the AI robot may be worn by a human to assist an operation of the human.

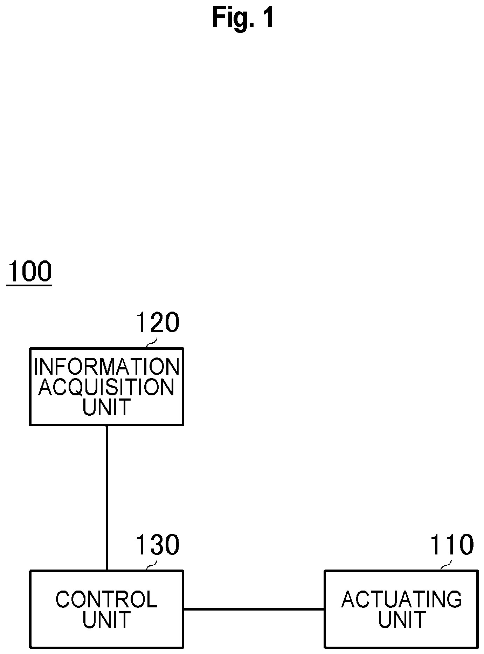

Fig. 1 schematically shows an example of a control device 100 according to the present embodiment. The control device 100 is an example of a control device of the AI robot. The control device 100 includes an actuating unit 110, an information acquisition unit 120, and a control unit 130.

The actuating unit 110 may be a member that moves an object. The actuating unit 110 may be a robot. The actuating unit 110 may be a robot arm. The actuating unit 110 may be arbitrarily member as long as it can move an object. The actuating unit 110 may be a member that is worn by a human and assists a motion of the human.

The information acquisition unit 120 may be an example of the ultra-high performance information acquisition unit. The information acquisition unit 120 may acquire information by sensors mounted on all parts of the robot, such as a fingertip, an eye, and a head of the robot. The information acquisition unit 120 may acquire information from sensors attached to tips of fingertips of ten fingers of the robot. The information acquisition unit 120 may acquire information from a sensor attached to a first joint of the finger of the robot. The information acquisition unit 120 may acquire information from a sensor attached to a second joint of the finger of the robot. The information acquisition unit 120 may acquire information from the sensors attached to the first joint and the second joint of the finger of the robot.

Examples of the sensor include a camera, a highest-performance camera, a solid-state LiDAR, a multi-color laser coaxial displacement meter, and the like, but are not limited thereto, and various other sensors may be used. Examples thereof include a sound collector, an odor detector, a vibratory meter, a thermo camera, a hardness meter, a radar, LiDAR, a high-pixel/telephoto/ultra-wide angle/360 degrees/high-performance camera, and sensors of vision recognition, fine sound, ultrasonic wave, vibration, infrared ray, ultraviolet ray, electromagnetic wave, temperature, humidity, spot AI weather forecast, high-accuracy multi-channel GPS, low-altitude satellite information, and long tail incident AI data. The long tail incident AI data may be trip data of an automobile with level 5 implementation.

Examples of the sensor information acquired from the plurality of types of sensors include an image, a distance, vibration, heat, an odor, a color, a sound, an ultrasonic wave, an ultraviolet ray, an infrared ray, and the like. Other examples include a taste, an odor, a shape, a temperature, a color, hardness, softness, and the like of a dish. Other examples include a motion of surgery performed by a doctor, a type of surgical instrument used, patient information, medicine information, and the like. Other examples include a motion of a factory worker, tools used, know-how of a worker, skills of a master, an assembly order, a method of making an article, and the like. Other examples include movement of the center of gravity of the weight, detection of a material of a road, detection of an outside air temperature, detection of an outside air humidity, detection of a vertical and lateral oblique inclination angle of a slope, a degree of freezing of a road, detection of a moisture content, a material of each tire, a wear state, detection of an air pressure, a road width, presence or absence of overtaking prohibition, vehicle type information of an oncoming vehicle and front and rear vehicles, a cruising state of those vehicles, a surrounding situation (birds, animals, soccer balls, accident vehicles, earthquakes, housework, winds, typhoons, heavy rain, light rain, snowstorm, fog, and the like), and the like. In the present embodiment, the various sensors perform these detections every nanosecond, and the information acquisition unit 120 acquires these pieces of information every nanosecond.

The control unit 130 controls the actuating unit 110 by using the information acquired by the information acquisition unit 120. The control unit 130 may control the actuating unit 110 by using a plurality of pieces of sensor information acquired by the information acquisition unit 120 from the plurality of sensors. The control unit 130 may acquire information according to a purpose from the information acquisition unit 120 and control the actuating unit 110 according to the purpose. The control unit 130 may acquire various types of information from the information acquisition unit 120 and control the actuating unit 110 by using information according to the purpose among the acquired information.

The control unit 130 may control the actuating unit 110 by using the information acquired by the information acquisition unit 120 and AI. The control unit 130 may control the actuating unit 110 in units of 1/1 billion seconds by using the information acquired by the information acquisition unit 120 and AI.

The information acquisition unit 120 and the control unit 130 may execute accurate image recognition, distance recognition, vibration recognition, heat recognition, odor recognition, color recognition, sound recognition, ultrasonic wave recognition, ultraviolet ray recognition, infrared ray recognition, and the like every nanosecond.

For example, the control unit 130 controls the actuating unit 110 by using the information acquired by the information acquisition unit 120 and AI, and the actuating unit 110 drives a vehicle. For example, the actuating unit 110 is worn by a human, and the control unit 130 causes the actuating unit 110 to assist a motion of a human who drives a vehicle by using the information acquired by the information acquisition unit 120 and AI.

As described above, the information acquisition unit 120 may acquire at least one of heat, a shape, a color, a texture, and an odor of a dish as information. The control unit 130 may control the actuating unit 110 by using the information acquired by the information acquisition unit 120 and AI, and cause the actuating unit 110 to reproduce a dish. Furthermore, the actuating unit 110 may be worn by a human, and the control unit 130 may cause the actuating unit 110 to assist a motion of a human who cooks by using the information acquired by the information acquisition unit 120 and AI. In the control device 100, once the highest performance of a chef having the highest skill is analyzed and recognized with ultra-high definition, the taste of the chef can be reproduced more accurately more than the chef himself/herself and repeatedly.

As described above, the information acquisition unit 120 may acquire an operation of surgery of a doctor as information. The control unit 130 may control the actuating unit 110 by using the information acquired by the information acquisition unit 120 and AI and cause the actuating unit 110 to perform surgery. Furthermore, the actuating unit 110 may be worn by a human, and the control unit 130 may cause the actuating unit 110 to assist a motion of a human who performs surgery by using the information acquired by the information acquisition unit 120 and AI.

As described above, the information acquisition unit 120 may acquire an improved operation of a worker as information. The control unit 130 may control the actuating unit 110 by using the information acquired by the information acquisition unit 120 and AI and cause the actuating unit 110 to perform a factory work. Furthermore, the actuating unit 110 may be worn by a human, and the control unit 130 may cause the actuating unit 110 to assist a motion of a human who performs a factory work by using the information acquired by the information acquisition unit 120 and AI.

The AI robot realized by the control device 100 according to the present embodiment can be 10,000 times smarter than any human being. The actuating unit 110 may reproduce the same various motions as human fingers and arms by a multi-axis actuator, a high-performance motor, or the like. The actuating unit 110 may realize ultra-high performance speed control and accuracy that are superior to those of humans. The control device 100 calculates numerical values of perfect control of the number of motor spins of each of multiple axes, perfect course navigation, and perfect steering with the ultra-high performance AI compute capability, and realizes autonomous driving by ten fingers and arms. The material of the AI robot may be a silicon resin or the like and may be a flexible material having properties such as heat resistance, water resistance, light resistance, chemical resistance, and insulation, and having good grip. The AI robot may accurately acquire data such as a shape of an object of the operation and a distance and learn by AI.

According to the AI robot realized by the control device 100 according to the present embodiment, it is possible to realize a smart finger and arm that are moved by AI by itself, instead of being controlled by a human, with ultra-high performance for the first time in the human history, exceeding human's intelligence and ability by 10,000 times.

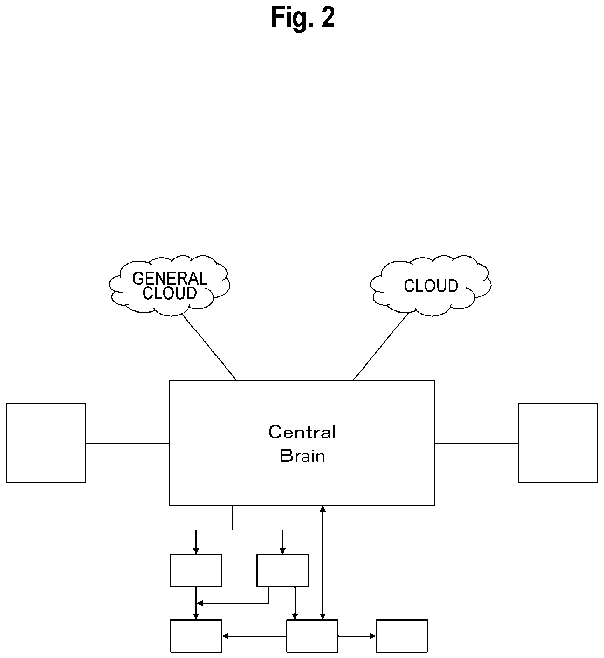

Fig. 2 schematically shows an example of a network configuration according to the present embodiment. A central brain may be an example of the control device 100.

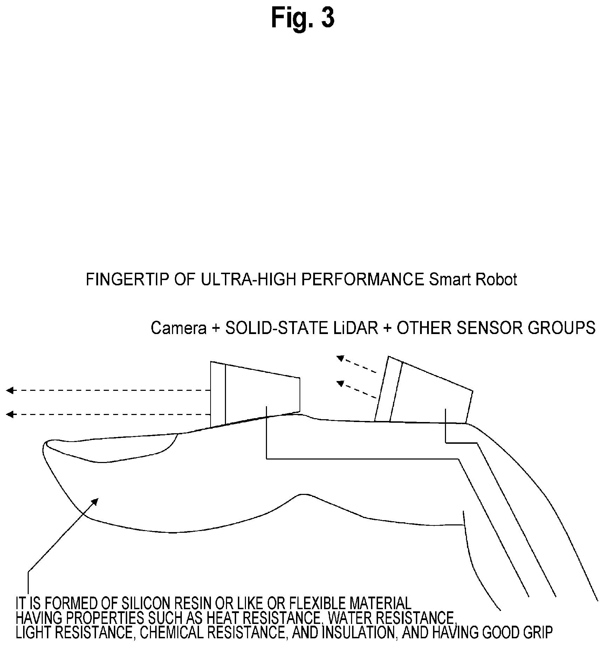

Fig. 3 schematically shows examples of sensors attached to a first joint and a second joint of a finger of a robot. The finger of the robot may be formed of a silicon resin or the like and may be formed of a flexible material having properties such as heat resistance, water resistance, light resistance, chemical resistance, and insulation, and also having good grip. The sensors of the first joint and the second joint may be a camera, a solid-state LiDAR, and other sensor groups. Further, the robot may have ten fingers like a human, and sensors may be attached to one or more finger joints.

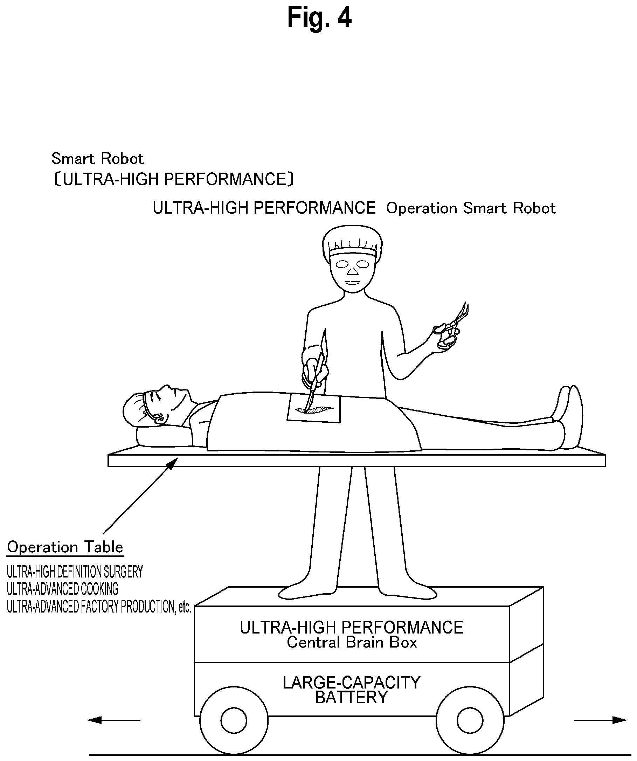

Fig. 4 schematically shows an example of an AI robot controlled by the control device 100. In the present example, the AI robot is disposed on a battery smart car equipped with an ultra-high performance central brain box, which is an example of the control device 100, and a large-capacity battery. The AI robot, and the central brain box and the large-capacity battery may be connected at a toe of the AI robot, so that the AI robot can also move a heel freely. The AI robot is controlled by the ultra-high performance central brain box and operates by using the power of the large-capacity battery. The AI robot can realize ultra-high definition surgery, ultra-advanced cooking, ultra-high performance factory production, and the like.

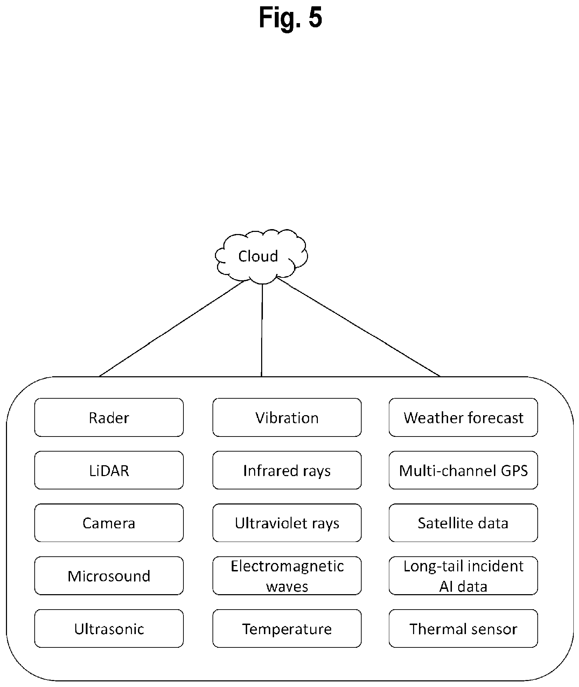

Fig. 5 schematically shows ultra-high performance information acquisition according to the present embodiment. In the present embodiment, a plurality of types of sensor information may be converted into AI data and accumulated in the cloud. The control device 100 may use cloud data.

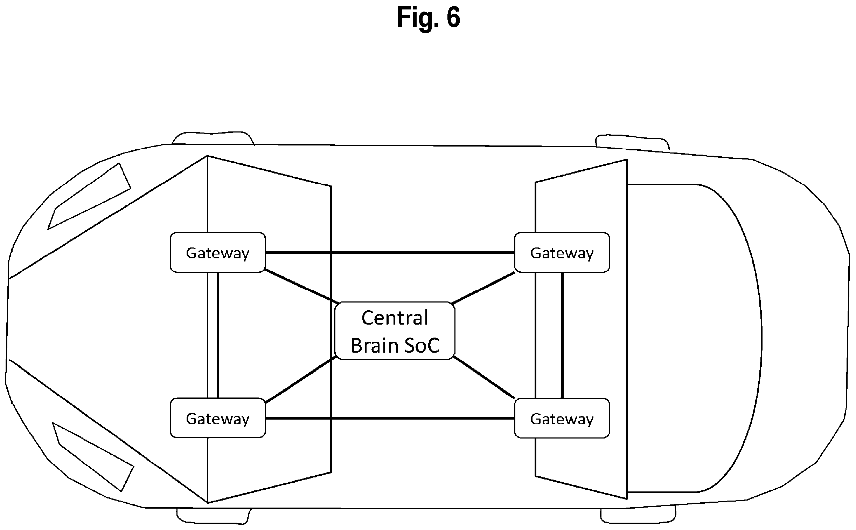

Fig. 6 schematically shows ultra-high performance automatic driving realized by the AI robot according to the present embodiment. The AI robot according to the present embodiment may be connected to a central brain SoC mounted on a vehicle via a network or may be directly connected thereto.

The control device 100 according to the present embodiment may execute matching with a weather forecast having the highest accuracy rate for each minimum spot by the entire road + AI from the acquired information. In addition, the control device 100 may execute matching with position information of another vehicle from the acquired information. Furthermore, the control device 100 may execute matching (matching of battery remaining amount with the speed for every nanosecond on the route) with a best estimated vehicle type from the acquired information. Furthermore, the control device 100 may execute matching with a mood of music or the like that a passenger is listening to from the acquired information. In addition, the control device 100 may execute instantaneous condition rearrangement in which a desired mood is changed from the acquired information.

The central brain SoC may upload AI data to the cloud at the time of vehicle charging, for example. A data lake is formed and the AI analyzes and always uploads to the latest state.

The control device 100 may use both software and hardware as a method of optimizing a passage of a vehicle. In terms of software, the control device 100 uses AI to best mix all the cloud accumulated information and the sensor information of the automobile, and the AI makes decisions every nanosecond to realize automatic driving that meets the demand of the passenger. In terms of hardware, the vehicle microcontrols a rotational output of a motor every 1/1 billion seconds (nanoseconds). The vehicle includes electricity and a motor capable of communicating and controlling in nanoseconds. According to the control device 100, since the AI predicts a crisis, it is possible to perform perfect stop without the need for braking and without spilling a cup of water. In addition, power consumption is low, and brake friction does not occur.

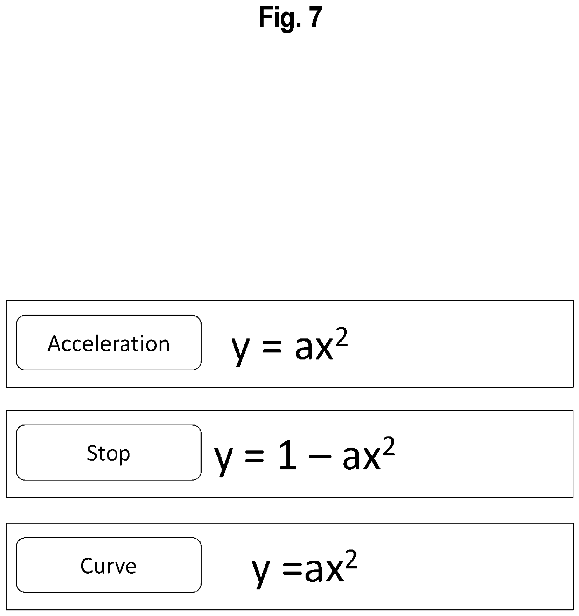

Fig. 7 schematically shows perfect speed control realized by control by the control device 100 according to the present embodiment. The principle shown in Fig. 7 serves as an index for calculating a braking distance of the vehicle, and is controlled by this basic equation. In the system according to the present embodiment, since there is ultra-high performance input data, calculation can be performed with a clean bell curve.

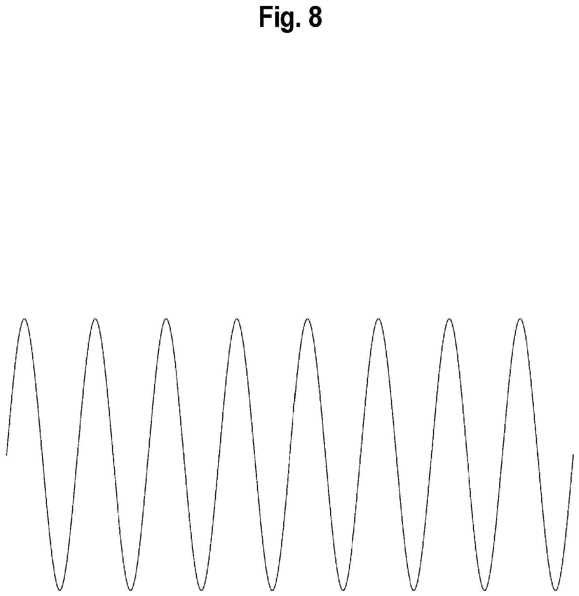

Fig. 8 schematically shows perfect bell curves realized by the control by the control device 100 according to the present embodiment.

The calculation speed when the ultra-high performance automatic driving is realized can be realized by 1Million TOPS.

As described above, in the present embodiment, the control device 100 may realize perfect cruise control. The brain of the control device 100 may execute control according to a desire of an occupant in the vehicle. As examples of the desire of the occupant, there are "shortest time", "longest battery remaining amount", "most want to avoid carsickness", "most want to feel G (safely)", "most want to feel a landscape by the mix of the above and the like", "most want to feel a landscape different from the previous one", "for example, want to follow memories of the road I came with someone a few years ago", "most want to avoid a probability of an accident", and the like, and the brain consults with the passenger about other various conditions, and the brain executes perfect mixing with the vehicle by the number of passengers, weight, position, movement of the center of gravity of the weight (calculated every nanosecond), detection of the material of the road every nanosecond, detection of the outside air humidity every nanosecond, detection of the outside air humidity every nanosecond, and selection of the total condition every nanosecond.

The brain may take into consideration and execute things such as "upper, lower, lateral, oblique inclination angle of a road slope", "matching with a weather forecast having the highest accuracy rate for each minimum spot by the entire route + AI", "matching with position information of another vehicle every nanosecond", "matching of those with a best estimated vehicle type (matching of a battery remaining amount with the speed for every nanosecond on the route)", "matching with a mood of music or the like that the passenger is listening to", "instantaneous condition rearrangement in which a desired mood is changed", "estimation of the optimal mix of a degree of freezing of a road every nanosecond, moisture content, wear of the material of each tire, such as 4, 2, 8, and 16 tires, air pressure, and the rest of the road", "the lane width and angle, and whether or not overtaking is prohibited on the road at that time", "type of vehicle in opposed-lane and forward and rear lane and the cruising state of the vehicle (for every nanosecond)", and "best mix of all other conditions".

The places to take, not just the center, within the width of each lane are all different. It varies depending on the speed, angle, and road information at that time. For example, inference matching of the best probability of influences every nanosecond for flying birds, animals, oncoming vehicles, flying soccer balls, children, accident vehicles, earthquakes, fires, winds, typhoons, heavy rain, light rain, snowstorm, fog, and others is executed.

The most recently updated perfect matching of those with the capability of the version of brain at that time point and the brain cloud accumulated by that time point is executed.

This may be defined as perfect cruising of ultra-high performance automatic driving. Therefore, for the ultra-high performance automatic driving, the best power management of the battery at that time point and the AI synchronized burst chilling function of temperature are required for the 1million TOPS.

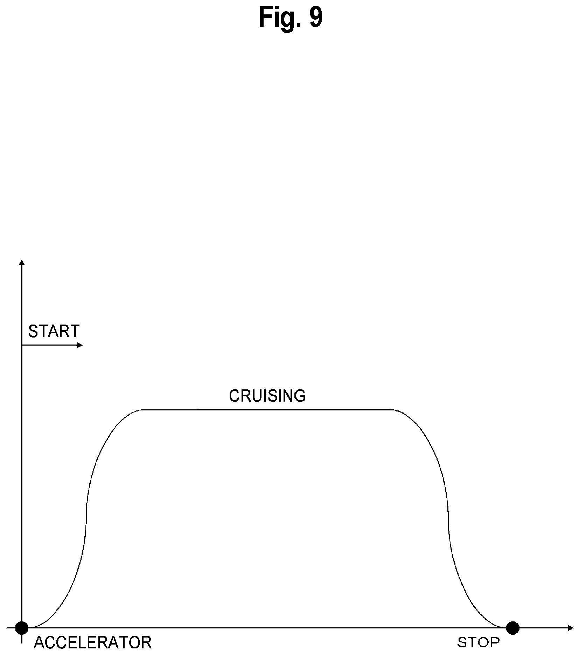

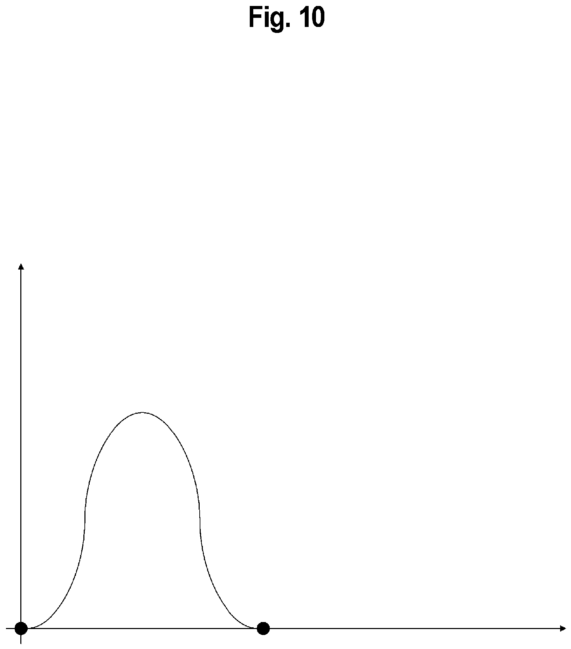

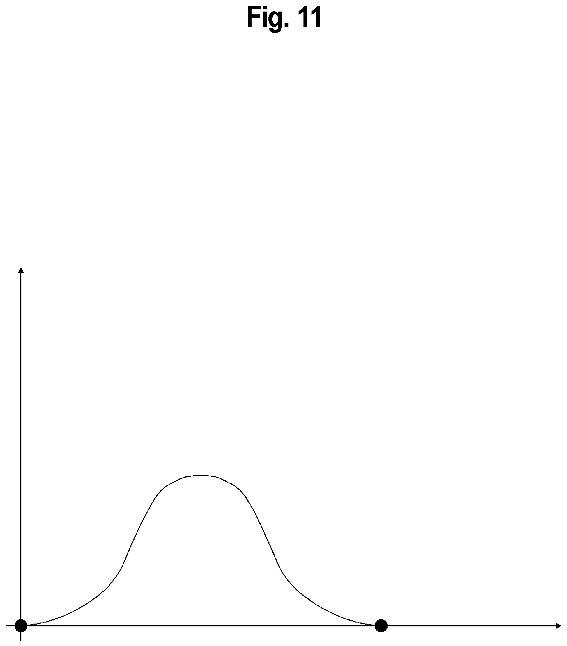

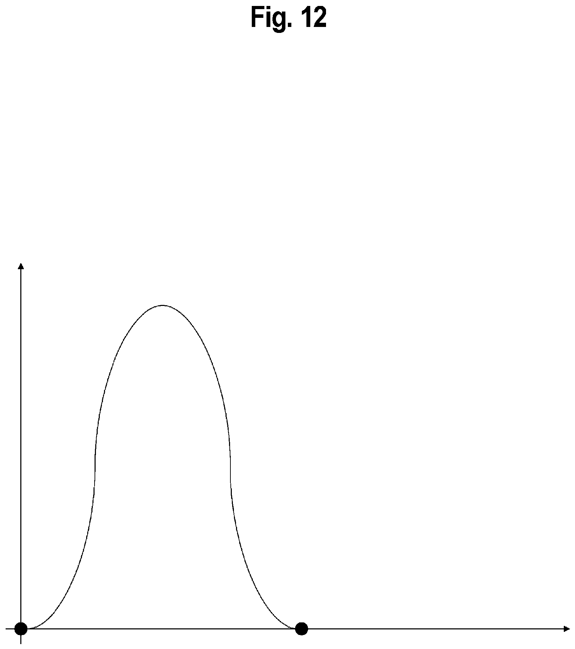

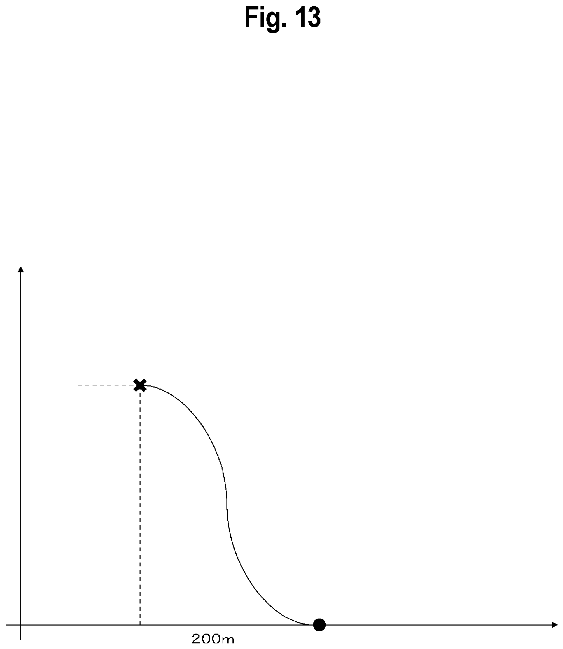

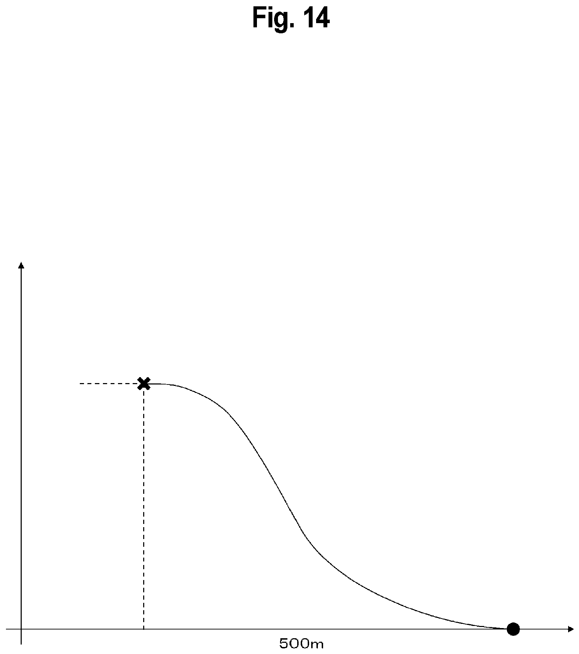

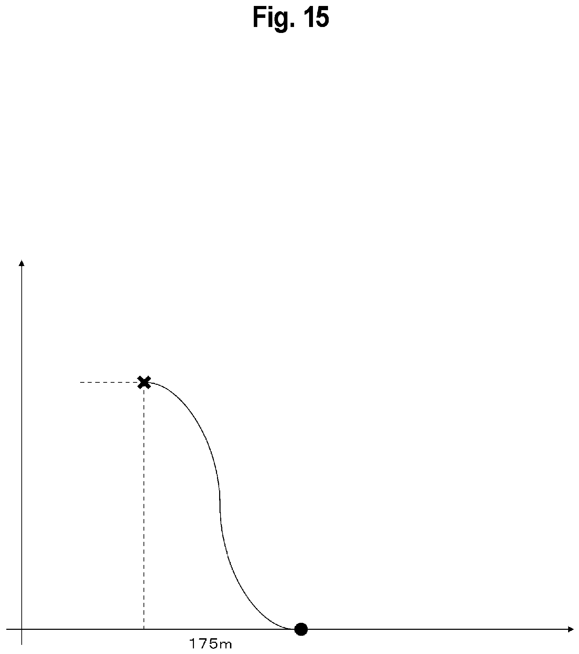

Figs. 9, 10, 11, 12, 13, 14, and 15 are schematic views of the perfect cruising.

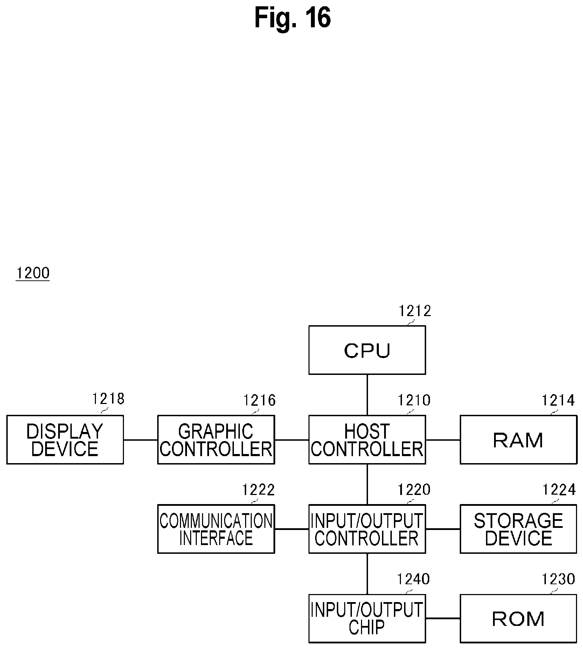

Fig. 16 schematically shows an example of a hardware configuration of a computer 1200 functioning as the control device 100 or the central brain SoC. The program installed in the computer 1200 can cause the computer 1200 to function as one or more "units" of the device according to the present embodiment, or cause the computer 1200 to execute an operation or the one or more "units" associated with the device according to the present embodiment, and/or cause the computer 1200 to execute a process or a stage of the process according to the present embodiment. Such a program may be executed by a CPU 1212 to cause the computer 1200 to execute certain operations associated with some or all of the blocks in the flowcharts and block views described herein.

The computer 1200 according to the present embodiment includes the CPU 1212, a RAM 1214, and a graphic controller 1216, which are mutually connected by a host controller 1210. The computer 1200 also includes input/output units such as a communication interface 1222, a storage device 1224, a DVD drive, and an IC card drive, which are connected to the host controller 1210 via an input/output controller 1220. The DVD drive may be a DVD-ROM drive, a DVD-RAM drive, or the like. The storage device 1224 may be a hard disk drive, a solid state drive, or the like. The computer 1200 also includes a ROM 1230 and a legacy input/output unit such as a keyboard, which are connected to the input/output controller 1220 via an input/output chip 1240.

The CPU 1212 operates according to programs stored in the ROM 1230 and the RAM 1214, thereby controlling each unit. The graphic controller 1216 obtains image data generated by the CPU 1212 in a frame buffer or the like provided in the RAM 1214 or itself, and causes the image data to be displayed on a display device 1218.

The communication interface 1222 communicates with other electronic devices via a network. The storage device 1224 stores programs and data used by the CPU 1212 in the computer 1200. The DVD drive reads programs or data from the DVD-ROM or the like and provides the programs or data to the storage device 1224. The IC card drive reads programs and data from an IC card and/or writes the programs and data to the IC card.

The ROM 1230 stores therein a boot program executed by the computer 1200 at the time of activation and/or a program that depends on hardware of the computer 1200. The input/output chip 1240 may also connect various input/output units to the input/output controller 1220 via a USB port, a parallel port, a serial port, a keyboard port, a mouse port, or the like.

The programs are provided by a computer-readable storage medium such as a DVD-ROM or an IC card. The programs are read from the computer-readable storage medium, installed in the storage device 1224, the RAM 1214, or the ROM 1230, which is also an example of the computer-readable storage medium, and executed by the CPU 1212. The information processing described in these programs is read by the computer 1200 and provides cooperation between the programs and the various types of hardware resources. The device or method may be configured by realizing an operation or processing of information according to the use of the computer 1200.

For example, when communication is executed between the computer 1200 and an external device, the CPU 1212 may execute a communication program loaded in the RAM 1214 and instruct the communication interface 1222 to execute communication processing based on the processing described in the communication program. Under the control of the CPU 1212, the communication interface 1222 reads transmission data stored in a transmission buffer area provided in the recording medium such as the RAM 1214, the storage device 1224, a DVD-ROM, or an IC card, transmits the read transmission data to the network, or writes reception data received from the network to a reception buffer area or the like provided on the recording medium.

In addition, the CPU 1212 may cause the RAM 1214 to read all or a necessary portion of a file or database stored in the external recording medium such as the storage device 1224, a DVD drive (DVD-ROM), an IC card, or the like, and may execute various types of processing on data on the RAM 1214. Next, the CPU 1212 may write back the processed data to the external recording medium.

Various types of information such as various types of programs, data, tables, and databases may be stored in a recording medium and subjected to information processing. The CPU 1212 may execute various types of processing on the data read from the RAM 1214, including various types of operations, information processing, condition determination, conditional branching, unconditional branching, information search/replacement, and the like, which are described throughout the present disclosure and designated by a command sequence of a program, and writes back the results to the RAM 1214. In addition, the CPU 1212 may search for information in a file, a database, or the like in the recording medium. For example, in a case where a plurality of entries each having an attribute value of a first attribute associated with an attribute value of a second attribute is stored in the recording medium, the CPU 1212 may search for an entry in which the attribute value of the first attribute matches a designated condition from the plurality of entries, and read the attribute value of the second attribute stored in the entry, thereby acquiring the attribute value of the second attribute associated with the first attribute satisfying the predetermined condition.

The program or software module described above may be stored in a computer-readable storage medium on the computer 1200 or in the vicinity of the computer 1200. Furthermore, a recording medium such as a hard disk or a RAM provided in a server system connected to a dedicated communication network or the Internet can be used as a computer-readable storage medium, thereby providing programs to the computer 1200 via the network.

The blocks in the flowcharts and block views in the present embodiment may represent stages of a process in which an operation is executed or "units" of a device that are responsible for executing the operation. Certain stages and "units" may be implemented by a dedicated circuit, a programmable circuit provided with computer-readable instructions stored on a computer-readable storage medium, and/or a processor provided with computer-readable instructions stored on a computer-readable storage medium. The dedicated circuit may include a digital and/or analog hardware circuit, and may include an integrated circuit (IC) and/or a discrete circuit. The programmable circuit may include a reconfigurable hardware circuit including, for example, logical products, disjunction, exclusive disjunction, NAND, NOR, and other logical operations, flip-flops, registers, and memory elements, such as field programmable gate arrays (FPGA) and programmable logic arrays (PLA).

A computer-readable storage medium may include arbitrarily tangible device capable of storing instructions for execution by a suitable device, such that a computer-readable storage medium having instructions stored thereon includes an article of manufacture including instructions that may be executed to create means for executing the operations designated in the flowcharts or block views. Examples of the computer-readable storage medium may include an electronic storage medium, a magnetic storage medium, an optical storage medium, an electromagnetic storage medium, a semiconductor storage medium, and the like. More specific examples of the computer-readable storage medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disk (DVD), a Blu-Ray (registered trademark) disk, a memory stick, an integrated circuit card, and the like.

The computer-readable instructions may include either a source code or an object code described in arbitrarily combination of one or more programming languages, including assembler instructions, instruction-set-architecture (ISA) instructions, machine instructions, machine-dependent instructions, microcode, firmware instructions, state-setting data, or object oriented programming languages such as Smalltalk (registered trademark), JAVA (registered trademark), C++, or the like, and conventional procedural programming languages, such as the "C" programming language or similar programming languages.

The computer-readable instructions may be provided for a processor of a general purpose computer, a special purpose computer, or other programmable data processing devices, or a programmable circuit, either locally or over a wide area network (WAN), such as a local area network (LAN), the Internet, or the like, to cause the processor or programmable circuit of the general purpose computer, the special purpose computer, or the other programmable data processing devices to execute the computer-readable instructions to generate means for the processor or programmable circuit to execute the operations designated in the flowcharts or block views. Examples of the processor include a computer processor, a processing unit, a microprocessor, a digital signal processor, a controller, a microcontroller, and the like.

### (2) Second Embodiment

JP 2022-061761 A discloses a technique for allowing a robot to reproduce a complicated operation performed by a human. An object of the invention disclosed in a second embodiment is to provide a technique for reproducing the skills of a master in accordance with the application.

An operation reproducing device according to the second embodiment includes an actuating unit that reproduces an operation of a model; an information acquisition unit that acquires information related to the operation of the model from a sensor; a designated item acquisition unit that acquires a designated item when reproducing the operation of the model; and a control unit that causes the actuating unit to reproduce the operation based on the information acquired from the sensor and the designated item.

An operation reproducing method according to the second embodiment, executed by a computer, the computer functioning as**:** an information acquisition unit that acquires information related to an operation of a model from a sensor, and a designated item acquisition unit that acquires a designated item when reproducing the operation of the model, the method includes: a step of causing an actuating unit that reproduces an operation of a model to reproduce the operation based on the information acquired from the sensor and the designated item.

According to the present invention, it is possible to provide a technique for reproducing the skills of a master according to the application.

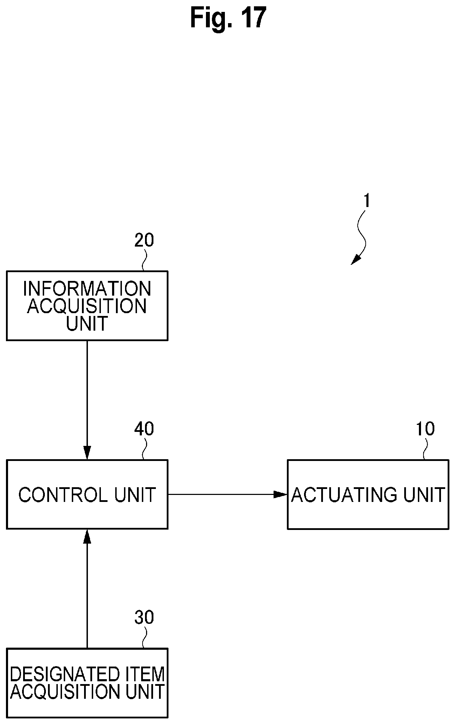

Fig. 17 is a view showing a configuration of an operation reproducing device 1 according to the second embodiment. The operation reproducing device 1 is configured by one computer or a plurality of computers connected via a communication line. As shown in Fig. 17, the operation reproducing device 1 includes an actuating unit 10, an information acquisition unit 20, a designated item acquisition unit 30, and a control unit 40.

The actuating unit 10 is, for example, a member that performs a work, such as a robot or a robot arm, or a member that is worn on a hand, a foot, or the like of a human to assist a motion of a human. The robot can make a dish on behalf of a first-class chef or play on behalf of an athlete. In a case where the actuating unit 10 is worn by a human, an operation can be assisted so that a person wearing the actuating unit can make a dish like a first-class chef or play like an athlete.

It is desirable that the actuating unit 10 can reproduce the same various motions as human fingers and arms by a multi-axis actuator, a high-performance motor, or the like. In addition, it is desirable that the actuating unit 10 can realize ultra-high performance speed control and accuracy superior to those of a human.

The information acquisition unit 20 acquires information by sensors mounted on all parts of the robot, such as a fingertip, an eye, and a head of an operation model. The operation model may be a human such as a first-class chef or athlete, or may be a robot that realizes an accurately controlled operation. Examples of the sensor include a camera, a solid-state LiDAR, a multi-color laser coaxial displacement meter, a sound collector, an odor detector, a vibratory meter, a thermo camera, a hardness meter, a radar, LiDAR, a high-pixel/telephoto/ultra-wide angle/360 degrees/high-performance camera, and sensors of vision recognition, fine sound, ultrasonic wave, vibration, infrared ray, ultraviolet ray, electromagnetic wave, temperature, humidity, spot AI weather forecast, high-accuracy multi-channel GPS, low-altitude satellite information, and long tail incident AI data. The long tail incident AI data is trip data of an automobile with level 5 implementation.

Examples of the information acquired from the sensor include an image, a distance, vibration, heat, an odor, a color, a sound, an ultrasonic wave, an ultraviolet ray, an infrared ray, and the like. Other examples include a taste, an odor, a shape, a temperature, a color, hardness, softness, and the like of a dish. Other examples include movement of the center of gravity of the weight, detection of a material of a ground, detection of an outside air temperature, detection of an outside air humidity, detection of a vertical and lateral oblique inclination angle of a slope, and a surrounding situation (birds, animals, soccer balls, accident vehicles, earthquakes, housework, winds, typhoons, heavy rain, light rain, snowstorm, fog, and the like), and the like. In the present embodiment, the various sensors may detect these pieces of information every nanosecond, and the information acquisition unit 20 may acquire these pieces of information every nanosecond.

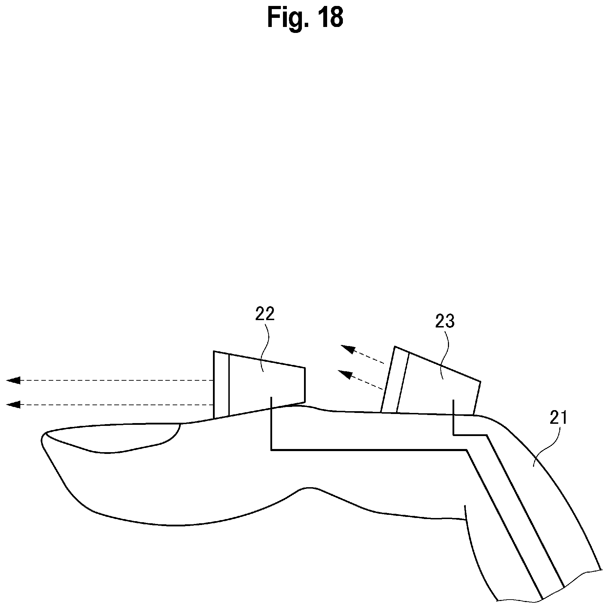

The information acquisition unit 20 may include a sensor 22 attached to a first joint of a finger 21 and a sensor 23 attached to a second joint as shown in Fig. 18. The finger 21 may be a robot finger, and in this case, the finger 21 may be formed of, for example, a silicon resin, and may be formed of a flexible material having properties such as heat resistance, water resistance, light resistance, chemical resistance, and insulation, and also having good grip. The sensors 22 and 23 of the first joint and the second joint may include a camera, a solid-state LiDAR, and other sensor groups. Further, the robot may have ten fingers 21 like a human, and sensors may be attached to one or more finger joints. Note that the information acquired by the information acquisition unit 20 may be converted into AI data and accumulated in the cloud.

The designated item acquisition unit 30 receives designation of a condition when the actuating unit 10 reproduces an operation or assists a motion of a human. A content to be designated may be directly input via the input means such as various operation buttons or a touch panel. For example, in a case of reproducing a dish, a taste preference, a scheduled completion time, and the like may be designated. In addition, the actuating unit 10 may feed back information related to a motion of a human wearing the actuating unit to the designated item acquisition unit 30, and the designated item acquisition unit 30 may acquire the fed back information as a condition. For example, in a case of assisting cooking, a speed and a difficulty level of cooking may be set as conditions in accordance with the skill of a human to be worn.

The control unit 40 may be a general-purpose computer, may be configured by one computer, or may be configured by a plurality of computers distributed on a communication network N**.** The control unit 40 includes a CPU, a memory such as a ROM and a RAM, an input interface, an output interface, a communication interface, a bus connecting these, and the like, and realizes various functions by the CPU executing a program stored in the ROM or the like.

The control unit 40 controls the actuating unit 10 based on the information acquired by the information acquisition unit 20. For example, the control unit 40 may control the actuating unit 10 by using the information acquired by the information acquisition unit 20 and artificial intelligence (AI). More specifically, the control unit 40 may control the actuating unit 10 in units of 1/1 billion seconds by using the information acquired by the information acquisition unit 20 and AI. The information acquisition unit 20 and the control unit 40 may execute accurate image recognition, distance recognition, vibration recognition, heat recognition, odor recognition, color recognition, sound recognition, ultrasonic wave recognition, ultraviolet ray recognition, infrared ray recognition, and the like every nanosecond.

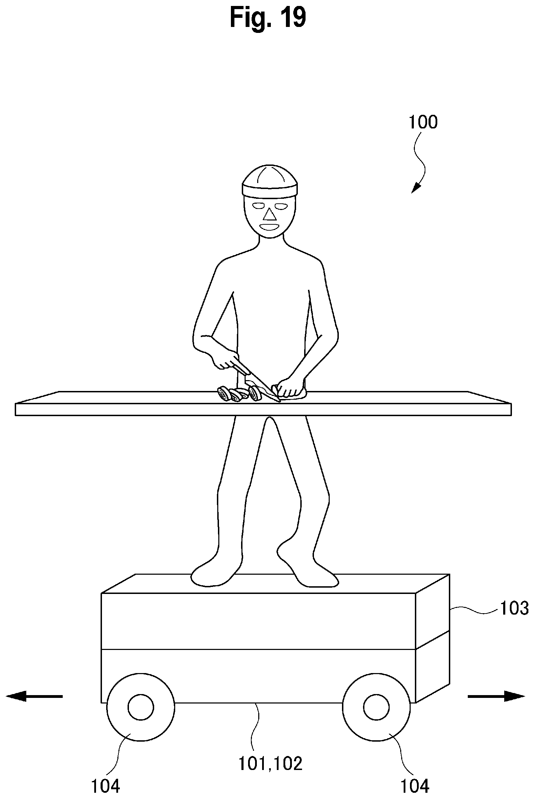

Fig. 19 is a view showing an example of a robot 100 (actuating unit 10) realized by the operation reproducing device 1**.** As shown in Fig. 19, the robot 100 is disposed on a table 103 on which a computer 101 (control unit 40) and a large-capacity battery 102 are mounted. The table 103 is configured to be movable by wheels 104. The robot 100 may be connected to the computer 101 and the large-capacity battery 102 at a toe portion 105. This allows the robot 100 to move freely including a heel portion. The robot 100 operates using the power of the large-capacity battery 102 under the control of the computer 101. The robot 100 can realize operations such as advanced cooking and sports.

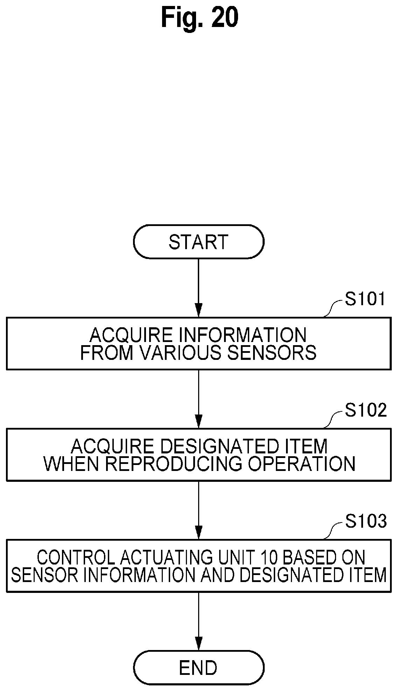

Fig. 20 is a flowchart showing an outline of an operation of the operation reproducing device 1**.**

First, the information acquisition unit 20 acquires information related to an operation of a model via various sensors (step S101). Note that the information acquired from the various sensors may be converted into AI data and stored in the cloud or the like. Next, the designated item acquisition unit 30 acquires a designated item when reproducing the operation of the model (step S102). When there is no designated item, the process proceeds from step S101 to step S103. In step S103, the control unit 40 controls the actuating unit 10 based on the information acquired from the various sensors and the designated item. When there is no designated item, the actuating unit 10 is controlled based on the information acquired from the various sensors.

### (Application to Cooking)

An example in which the operation reproducing device 1 according to the present embodiment is applied to reproduction of a dish will be specifically described. As described above, the information acquisition unit 20 may acquire at least one of heat, a shape, a color, a texture, and an odor of a dish as information. In addition, the control unit 40 may control the actuating unit 10 by using the information acquired by the information acquisition unit 20 and AI and cause the actuating unit 10 to reproduce a dish. Furthermore, in a case where the actuating unit 10 is worn by a human, the control unit 40 may cause the actuating unit 10 to assist a motion of a human who cooks by using the information acquired by the information acquisition unit 20 and AI. In the operation reproducing device 1, once the highest performance of a chef having the highest skill is analyzed and recognized with ultra-high definition, the taste of the chef can be reproduced more accurately more than the chef himself/herself and repeatedly.

In the case of being applied to reproduction of a dish, the information acquisition unit 20 acquires, via various sensors, various types of information related to a cooking process, such as information related to a motion of a hand and a line of sight when a first-class chef cooks (operation of a cook), information related to a time taken for each cooking process, a shape and a size (how to cut) of an ingredient, a fire level, and a temperature, a color, and an odor of a dish. Based on the acquired information, the control unit 40 controls the robot arm and an auxiliary instrument (actuating unit 10) worn by the human so as to be able to reproduce the same dish as the dish cooked by the chef.

In addition, the control unit 40 may control the actuating unit 10 according to the designated condition (designated item) at the time of reproduction acquired by the designated item acquisition unit 30 together with the information acquired by the information acquisition unit 20. For example, when a time by which the dish is desired to be completed is designated, AI may inversely calculate a start time of each process and a speed of cooking in accordance with the designated time, and reflect the calculated start time and speed in the control of the actuating unit 10. Note that, in addition to directly designating the start time, a time to complete the dish may be automatically set according to a time when going out, a time when visitor comes, or the like in cooperation with a schedule management application or the like.

In addition, when a taste preference is designated, the taste of the dish of the chef may be finely adjusted according to the preference. For example, a preference of a strength of taste may be designated in five stages (weak, slightly weak, normal, slightly strong, and strong), and reproduction may be performed as it is in a case of normal, and the level of seasoning may be adjusted by AI in a case of weak or strong. A heating degree may be similarly adjusted. Furthermore, when the age of the person who eats is designated, AI may adjust the seasoning according to the age. For example, adjustment can be made such as making spiciness less for children or reducing a salt content for elderly people.

In addition, setting of a cook may be performed. When a cook is set to "chef", the taste of the chef is reproduced as it is, and for example, when the cook is set to "grandmother", AI may change the ingredients and seasoning, and a dish arranged more homely than the dish of the chef may be made.

In addition, in a case where the actuating unit 10 is an auxiliary instrument, the control may be changed according to the cooking skill of the person wearing the actuating unit. For example, a level of cooking (a professional cook, an ordinary person familiar with cooking, a person who does not usually cook, and the like) of the person wearing the actuating unit may be designated in advance, or the motion of the person wearing the actuating unit may be fed back to the designated item acquisition unit 30 via the actuating unit 10, and AI may determine the skill. The control unit 40 may reproduce a procedure similar to that of the chef in a case of a person with high skill, and may perform adjustment such as omitting or simplifying a difficult procedure in a case of an unfamiliar person. Furthermore, the motion of the person wearing the actuating unit and the motion of the chef (teacher data) may be compared to provide advice for improvement. For example, in a case where a way of holding a kitchen knife is different from that of the chef, a correct way of holding the kitchen knife may be presented by a voice or an image. As described above, the operation reproducing device 1 according to the present embodiment can also be used for learning a cooking technique.

### (Application to Sports)

The operation reproducing device 1 according to the present embodiment can also be applied to reproducing play of sports or assisting a motion of a human who plays, by using the actuating unit 10.

In the case of application to sports, the information acquisition unit 20 acquires, via various sensors, various types of information related to play of sports, such as information related to a body motion and a line of sight when a first-class athlete or a coach plays, and a moving speed. The control unit 40 controls an auxiliary instrument (actuating unit 10) worn on the robot or the human so as to reproduce the motion of the athlete or the coach based on the acquired information. In a case of a robot, it can be used as an opponent, a partner, a one-team player, or the like. In addition, in a case of being used as an auxiliary instrument, it can be used for learning of a technique and the like.

The control unit 40 may control the actuating unit 10 according to a condition designated at the time of reproduction acquired by the designated item acquisition unit 30 together with the information acquired by the information acquisition unit 20. For example, when a level (advanced person, intermediate person, beginner, and the like) of the opponent (robot) is set, a motion and speed may be adjusted according to the level designated by AI.

In addition, in a case where the actuating unit 10 is an auxiliary instrument, the control may be changed according to the skill of the person wearing the actuating unit. For example, a level (advanced person, intermediate person, beginner, and the like) of the person wearing the actuating unit may be designated in advance, or a motion of the person wearing the actuating unit may be fed back to the designated item acquisition unit 30 via the actuating unit 10, and AI may determine the skill. The control unit 40 may adjust the speed and intensity of the motion in accordance with the skill of the person wearing the actuating unit. Furthermore, the motion of the person wearing the actuating unit may be compared with the motion of the athlete or coach (teacher data), and a point to be improved may be advised by an image or a voice.

As described above, according to the present embodiment, various sensors are used to acquire information such as a motion of a first-class cook when cooking and a state of ingredients, and the AI robot reproduces the cooking operation of the cook based on the acquired information. Therefore, it is possible to accurately reproduce a dish made by a first-class chef.

Furthermore, at the time of reproducing the cooking, conditions regarding a taste preference and a completion time of a dish are designated, and AI adjusts the cooking operation in accordance with the designated conditions. Therefore, it is possible to reproduce a dish made by a first-class chef in accordance with the preferences and demands.

In addition, information of a motion and a line of sight when an athlete or a coach plays is acquired by using various sensors, and the AI robot reproduces an operation of the play based on the acquired information. Therefore, it is possible to accurately reproduce play of a skilled person such as an athlete or a coach.

Furthermore, at the time of reproducing the play, conditions regarding a level of the play is designated, and the motion is adjusted by AI according to the conditions, so that the play can be reproduced according to the level of the opponent or the participant.

In addition, since the motion of the person wearing the actuating unit 10 as the auxiliary instrument is fed back and the reproduction of the operation by the actuating unit 10 is adjusted in accordance with the motion of the person wearing the actuating unit, it is possible to reproduce the operation of the skilled person or the like within a reasonable range for the person to be assisted.

### (3) Third Embodiment

When a human can clearly grasp a difference between his/her own operation and an operation that serves as a model when learning a certain operation (for example, operations of cooking and sports, and the like), it is considered that an ideal operation can be efficiently learned, but such a technique has not yet been provided.

The invention disclosed in a third embodiment has been made in view of the above circumstances, and an object thereof is to provide an operation learning support device or the like which enables efficient learning of an operation that serves as a model.

An operation learning support device according to one aspect of the third embodiment is an operation learning support device that supports a user to learn an operation of a model, the operation learning support device including: an actuating unit that operates in accordance with given information; a first information acquisition unit that acquires, from a first sensor, information related to the operation of the model; a second information acquisition unit that acquires information related to an operation of the user via a second sensor worn by the user; an extraction unit that compares the information related to the operation of the model with the information related to the operation of the user and extracts a difference between the two; and a control unit that generates, based on the difference between the two, complementary information for complementing the difference in operation between the user and the model, and outputs the complementary information to the actuating unit.

According to the present invention, it is possible to efficiently learn an operation that serves as a model.

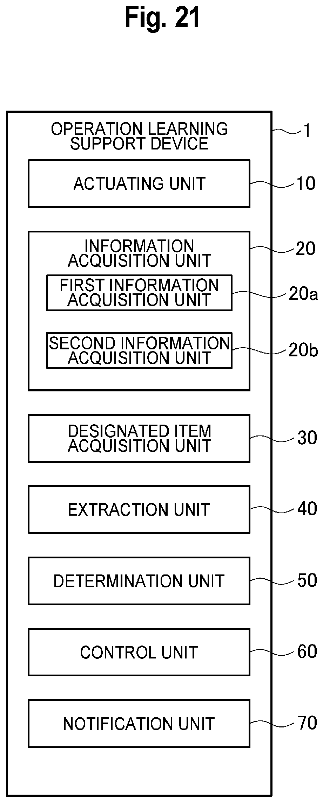

Fig. 21 is a view showing a configuration of an operation learning support device 1 according to the third embodiment. The operation learning support device 1 is configured by one computer or a plurality of computers connected via a communication line. As shown in Fig. 1, the operation learning support device 1 includes the actuating unit 10, the information acquisition unit 20, the designated item acquisition unit 30, an extraction unit 40, a determination unit 50, a control unit 60, and a notification unit 70. Note that the actuating unit 10, the information acquisition unit 20, the information acquisition unit 20, the designated item acquisition unit 30, the extraction unit 40, the determination unit 50, the control unit 60, and the notification unit 70 are realized by software installed in the operation learning support device 1 cooperating with hardware resources such as a CPU.

The actuating unit 10 is, for example, a wearable robot that is worn on a hand, a foot, or the like of a human to assist a motion of a human. The actuating unit 10 enables a person wearing the actuating unit (hereinafter, user) to make a dish like a first-class chef, or assists an operation of a user so as to play like a first-class athlete, by operating according to the given information. Furthermore, under the control of the control unit 60, the actuating unit 10 acquires information related to the operation of the user himself/herself wearing the actuating unit, and detects and feeds back a difference from a correct operation, thereby supporting the user to efficiently learn (that is, learning) the operation of a first-class chef, a first-class athlete, or the like.

It is desirable that the actuating unit 10 can reproduce the same various motions as human fingers and arms by a multi-axis actuator, a high-performance motor, or the like. In addition, it is desirable that the actuating unit 10 can realize ultra-high performance speed control and accuracy superior to those of a human.

The information acquisition unit 20 includes a first information acquisition unit 20a and a second information acquisition unit 20b. The first information acquisition unit 20a acquires information sensed by a sensor (first sensor) mounted on any part of a robot such as a fingertip, an eye, or a head of an operation model. The operation model (model) may be a human such as a first-class chef or athlete, or may be a robot that realizes an accurately controlled operation. The second information acquisition unit 20b acquires information sensed by a sensor (second sensor) worn by the user. In the following description, the first information acquisition unit 20a and the second information acquisition unit 20b are simply referred to as an information acquisition unit 20 in a case where it is not particularly necessary to distinguish them.

The example of each sensor and the example of information acquired from each sensor have already been described in the second embodiment, and thus, will be omitted here.

The information acquisition unit 20 may include the sensor 22 attached to a first joint of the finger 21 and the sensor 23 attached to a second joint as shown in Fig. 18 described above. The finger 21 may be a robot finger, and in this case, the finger 21 may be formed of, for example, a silicon resin, and may be formed of a flexible material having properties such as heat resistance, water resistance, light resistance, chemical resistance, and insulation, and also having good grip. The sensors 22 and 23 of the first joint and the second joint may include a camera, a solid-state LiDAR, and other sensor groups. Further, the robot may have ten fingers 21 like a human, and sensors may be attached to one or more finger joints. Of course, attachment positions of the sensors 22 and 23 are not intended to be limited to fingers, and can be applied to any part (for example, a foot, a shoulder, an arm, a waist, and the like). Note that the information acquired by the information acquisition unit 20 may be converted into AI data and accumulated in the cloud.

The designated item acquisition unit 30 receives designation of a condition when the actuating unit 10 reproduces an operation or assists a motion of a human. A content to be designated may be directly input via the input means such as various operation buttons or a touch panel. For example, in a case where the user learns an operation of a first-class chef, a type of dish, a recipe, a taste preference, a scheduled completion time, a speed of cooking, a difficulty level, and the like may be arbitrarily designated.

The extraction unit 40 compares the information related to the operation of the model acquired by the first information acquisition unit 20a with the information related to the operation of the user acquired by the second information acquisition unit 20b, and extracts a difference between the two.

The determination unit 50 determines an operation learning level of the user based on the difference between the two extracted by the extraction unit 40, and outputs a determination result. For example, in a case of learning an operation of a first-class chef, the determination unit 50 determines a learning level (for example, level 1 to level 10, and the like) by comparing the difference between the two with a threshold value. The threshold value may be arbitrarily set and changed by, for example, a person concerned who maintains and operates the operation learning support device 1. The person concerned can set arbitrary threshold value for various parameters (for example, a deviation amount and a number of times of deviation of the operation, and the like).

The control unit 60 generates and outputs complementary information for complementing the difference in operation between the user and the model based on the extracted difference between the two and the determination result of the operation learning level of the user.

For example, in the case of learning the operation of the first-class chef, the control unit 60 generates complementary information supporting how to use fingers or arms of the user wearing the actuating unit 10 so as to approach the kitchen knife handling by the first-class chef, and outputs the complementary information to the actuating unit 10 and the notification unit 70. At this time, the control unit 60 may generate the complementary information by changing a speed or a difficulty level of the kitchen knife handling according to the determination result of the learning level of the user. For example, in a case where the learning level of the user is low (level 1 to level 3), the control unit 60 may generate complementary information that slows the speed of kitchen knife handling or omits kitchen knife handling with a high difficulty level. The actuating unit 10 operates based on the complementary information given from the control unit 60, so that the user can efficiently learn the operation of the model (for example, a first-class chef or athlete).

Note that the control unit 60 may control the actuating unit 10 by using various types of information acquired by the information acquisition unit 20, artificial intelligence (AI), or the like, in addition to generating the complementary information. More specifically, the control unit 60 may control the actuating unit 10 in units of 1/1 billion seconds by using the information acquired by the information acquisition unit 20 and AI. The information acquisition unit 20 and the control unit 60 may execute accurate image recognition, distance recognition, vibration recognition, heat recognition, odor recognition, color recognition, sound recognition, ultrasonic wave recognition, ultraviolet ray recognition, infrared ray recognition, and the like every nanosecond.

The notification unit 70 is configured by, for example, a light emitting element such as an LED, a display device, a speaker, and the like, and notifies the user of the difference in operation when the difference between the two is extracted by the extraction unit 40. Specifically, the notification unit 70 notifies the difference (including occurrence of a difference, magnitude of a difference, and the like) in operation based on the complementary information supplied from the control unit 60. For example, in a case where a way of holding a kitchen knife of a user, a way of cutting ingredients, a way of mixing, and the like are different from those of a first-class chef, not only the operation of the user is supported by the actuating unit 10, but also a difference (that is, the difference in operation) from that of the first-class chef is presented to the user by a voice, an image, and the like by using the notification unit 70. As a result, the user can more objectively grasp the difference (difference) between the user's own operation and the operation of the model, and the efficiency of learning the operation of the model can be improved.

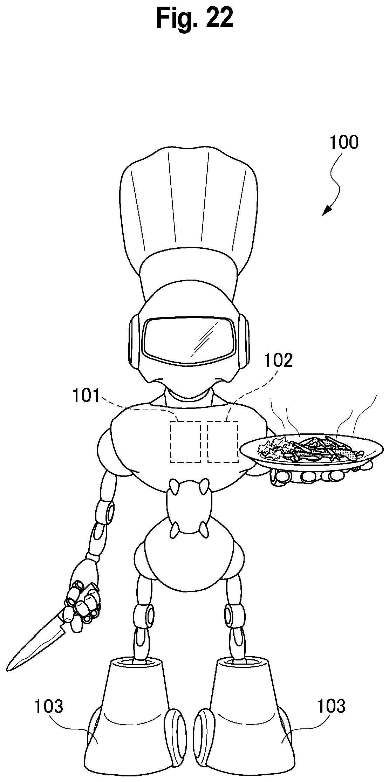

Fig. 22 is a view showing an example of the robot 100 realized by the operation learning support device 1. A case where it is applied to a humanoid cooking robot instead of a human is exemplified in Fig. 22. The robot 100 includes a computer 101, a large-capacity battery 102, wheels 103 for movement, and the like. The robot 100 can move freely. The robot 100 operates using the power of the large-capacity battery 102 under the control of the computer 101. Under the control of the computer 101, the robot 100 can faithfully reproduce, for example, an operation of a first-class chef that serves as a model. Note that software for faithfully reproducing operations of various models (for example, a first-class athlete and the like) may be installed in the robot 100, in addition to the first-class chef.

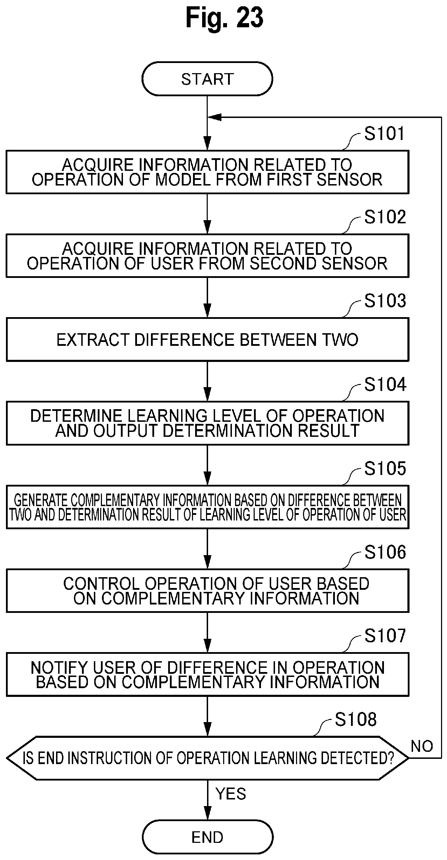

Fig. 23 is a flowchart showing an outline of the operation of the operation learning support device 1.

While the first information acquisition unit 20a acquires information related to the operation of the model via the first sensor (step S101), the second information acquisition unit 20b acquires information related to the operation of the user via the second sensor worn by the user (step S102). When the user or the like inputs a designated item when reproducing the operation of the model, the designated item acquisition unit 30 acquires the designated item. Thereafter, the extraction unit 40 compares the information related to the operation of the model acquired by the first information acquisition unit 20a with the information related to the operation of the user acquired by the second information acquisition unit 20b, and extracts a difference between the two (step S103).

The determination unit 50 determines an operation learning level of the user based on the difference between the two extracted by the extraction unit 40, and outputs a determination result (step S104). The control unit 60 generates complementary information for complementing the difference in operation between the user and the model based on the extracted difference between the two and the determination result of the operation learning level of the user (step S105), and outputs the complementary information to the actuating unit 10 and the notification unit 70. While the actuating unit 10 controls the operation of the user wearing the actuating unit 10 based on the complementary information (step S106), the notification unit 70 presents to the user that the difference in operation has occurred by a voice, an image, or the like based on the complementary information (step S107). In a case where an end instruction of the operation learning is not detected (step S108: NO), the operation learning support device 1 repeatedly executes the above-described series of processing. Thereafter, when the operation learning support device 1 detects an end instruction of the operation learning (step S108: YES), the operation learning support device 1 ends the processing described above.

### (Application to Cooking)

An example in which the operation learning support device 1 according to the present embodiment is applied to learning of an operation of a first-class chef will be specifically described. As described above, the information acquisition unit 20 may acquire at least one of heat, a shape, a color, a texture, and an odor of a dish as information. The information acquisition unit 20 acquires, via various sensors, various types of information related to a cooking process, such as information related to a motion of a hand and a line of sight when a first-class chef and the user cook (operation of a cook), information related to a time taken for each cooking process, a shape and a size (how to cut) of an ingredient, a fire level, and a temperature, a color, and an odor of a dish. The extraction unit 40 compares the information related to the operation of the first-class chef acquired by the information acquisition unit 20 with the information related to the operation of the user, and extracts a difference between the two. The control unit 60 controls an auxiliary instrument (actuating unit 10) or the like worn by the user so as to be able to reproduce the same dish as the dish cooked by the chef based on the difference between the two extracted by the extraction unit 40.

In addition, the control unit 60 may control the actuating unit 10 according to a designated condition (designated item) at the time of reproduction acquired by the designated item acquisition unit 30 together with the information acquired by the information acquisition unit 20. For example, when a time by which the dish is desired to be completed is designated, AI may inversely calculate a start time of each process and a speed of cooking in accordance with the designated time, and reflect the calculated start time and speed in the control of the actuating unit 10. Note that, in addition to directly designating the start time, a time to complete the dish may be automatically set according to a time when going out, a time when visitor comes, or the like in cooperation with a schedule management application or the like.

In addition, when a taste preference is designated, the taste of the dish of the chef may be finely adjusted according to the preference. For example, a preference of a strength of taste may be designated in five stages (weak, slightly weak, normal, slightly strong, and strong), and reproduction may be performed as it is in a case of normal, and the level of seasoning may be adjusted by AI in a case of weak or strong. A heating degree may be similarly adjusted. Furthermore, when the age of the person who eats is designated, AI may adjust the seasoning according to the age. For example, adjustment can be made such as making spiciness less for children or reducing a salt content for elderly people.

In addition, the user may be allowed to select (set) a cook that serves as a model. For example, when the user sets the cook to "chef", the taste of the chef is reproduced as it is, and when the user sets the cook to "grandmother", AI may change the ingredients and seasoning, and a dish arranged more homely than the dish of the chef may be made.

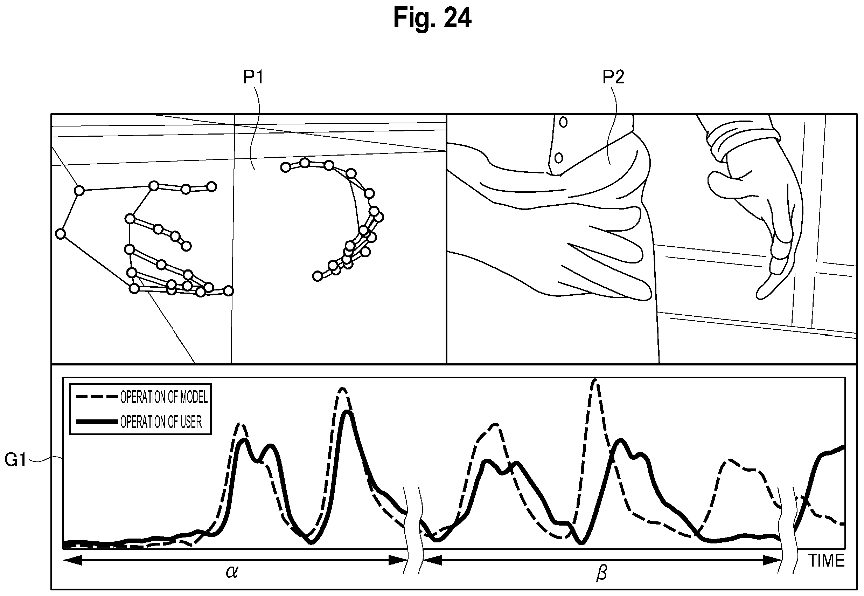

In addition, in a case where the actuating unit 10 is an auxiliary instrument, the control may be changed according to the cooking skill of the user wearing the actuating unit. For example, a level of cooking (a professional cook, an ordinary person familiar with cooking, a person who does not usually cook, and the like) of the user wearing the actuating unit may be designated in advance, or the motion of the user wearing the actuating unit may be fed back to the determination unit 50 via the actuating unit 10, and the determination unit 50 may determine the skill. The control unit 60 may perform adjustment such as omitting or simplifying a difficult procedure in a case where the skill of the user is low (that is, unfamiliar) while reproducing a procedure similar to that of the chef in a case where the skill of the user is high. For example, the following criteria can be used to determine which procedure is difficult and which procedure is simplified. As an example, an intense operation (for example, cutting into thin strips or the like) in which a motion related to cooking (speed, acceleration, or the like) exceeds a threshold value, or an operation in a section in which a difference between the operation of the model and the operation of the user is large (for example, pot shaking or the like) is recognized as a difficult procedure. Among the procedures determined to be difficult, which procedure is simplified or the like may be appropriately set or changed according to the skill of the user or the operation content. Note that the control unit 60 may compare the motion of the user wearing the actuating unit with the motion of the chef (teacher data) and present a comparison result to the user in an image, a graph, or the like. For example, as shown in Fig. 5, an image P1 indicating the motion of the user and an image P2 indicating the motion of the first-class chef may be displayed side by side on the display device, or a graph G1 indicating a difference between the motion of the user and the motion of the first-class chef (position, speed, acceleration, and the like) may be displayed on the display device. When the graph G1 shown in Fig. 24 is described as an example, a section α indicates an operation of finely cutting an ingredient (onion, carrot, or the like), and a section β indicates an operation of mixing and molding the ingredient (onion, minced meat, or the like). In the example shown in Fig. 5, since there is a large difference in operation (for example, a degree of force, and the like) between the model and the user (see section β), the user can objectively grasp how to improve the molding operation by confirming the graph G1 together with the images P1 and P2. In this manner, the user confirms the comparison result displayed on the display device, thereby further improving the efficiency of learning the operation of the first-class chef.

### (Application to Sports)

An example in which the operation learning support device 1 according to the present embodiment is applied to learning of an operation (play) of a first-class athlete will be described.

In the case of application to sports, the information acquisition unit 20 acquires, via various sensors, various types of information related to play of sports, such as information related to a body motion and a line of sight when a first-class athlete (including a coach or the like) plays, and a moving speed. The extraction unit 40 compares the information related to the play of the first-class athlete acquired by the information acquisition unit 20 with the information related to the play of the user, and extracts a difference between the two. The control unit 60 controls an auxiliary instrument (actuating unit 10) or the like worn by the user so as to be able to reproduce the play of the first-class athlete based on the difference between the two extracted by the extraction unit 40.

In addition, the control unit 60 may control the actuating unit 10 according to a condition designated at the time of reproduction acquired by the designated item acquisition unit 30 together with the information acquired by the information acquisition unit 20. For example, when a level (advanced person, intermediate person, beginner, and the like) of the opponent (robot) is set, a motion and speed may be adjusted according to the level designated by AI.

In addition, in a case where the actuating unit 10 is an auxiliary instrument, the control may be changed according to a skill (skill) of the user wearing the actuating unit. For example, a level (advanced person, intermediate person, beginner, and the like) of the user wearing the actuating unit may be designated in advance, or the motion of the user wearing the actuating unit may be fed back to the determination unit 50 via the actuating unit 10, and the determination unit 50 may determine the skill. The control unit 60 may adjust the speed and intensity of the motion in accordance with the skill of the user wearing the actuating unit. Furthermore, the motion of the user wearing the actuating unit and the motion of the athlete (teacher data) may be compared, and the comparison result may be presented to the user in an image, a graph, or the like.

As described above, according to the present embodiment, the difference between the operation of the user and the operation of the model is extracted by using each sensor, and the actuating unit worn by the user is controlled based on the extracted difference of the two. The user can grasp a difference (difference) between his/her own operation and the operation of the model via the actuating unit, and can efficiently learn the operation of the model.

Although the present invention has been described using the embodiments, the technical scope of the present invention is not limited to the scope described in the above embodiments. It is apparent to those skilled in the art that various modifications or improvements can be made to the above embodiments. It is apparent from the description of the claims that modes to which such changes or improvements are added can also be included in the technical scope of the present invention.

It should be noted that the order of execution of each processing such as operations, procedures, steps, and stages in the devices, systems, programs, and methods shown in the claims, the specification, and the drawings can be realized in arbitrarily order unless "before", "prior to", or the like is explicitly stated, and unless the output of the previous processing is used in the later processing. Even when the operation flow in the claims, the specification, and the drawings is described using "first,", "next,", and the like for convenience, it does not mean that it is essential to perform in this order.

Although the present invention has been described using the embodiments, the technical scope of the present invention is not limited to the scope described in the above embodiments. It is apparent to those skilled in the art that various modifications or improvements can be made to the above embodiments. It is apparent from the description of the claims that modes to which such changes or improvements are added can also be included in the technical scope of the present invention.

It should be noted that the order of execution of each processing such as operations, procedures, steps, and stages in the devices, systems, programs, and methods shown in the claims, the specification, and the drawings can be realized in arbitrarily order unless "before", "prior to", or the like is explicitly stated, and unless the output of the previous processing is used in the later processing. Even when the operation flow in the claims, the specification, and the drawings is described using "first,", "next,", and the like for convenience, it does not mean that it is essential to perform in this order.

### Reference Signs List

- 100: Control device
- 110: Actuating unit
- 120: Information acquisition unit
- 130: Control unit
- 1200: Computer
- 1210: Host controller
- 1212: CPU
- 1214: RAM
- 1216: Graphic controller
- 1218: Display device
- 1220: Input/output controller
- 1222: Communication interface
- 1224: Storage device
- 1230: ROM
- 1240: Input/output chip

## Claims

1. A control device comprising:
an actuating unit that moves an object;
an information acquisition unit that acquires a plurality of pieces of information; and
a control unit that controls the actuating unit by using the information acquired by the information acquisition unit and AI.

2. The control device according to claim 1, wherein the control unit controls the actuating unit in units of 1/1 billion seconds by using the information and AI, and the actuating unit drives a vehicle.

3. The control device according to claim 2, wherein
the actuating unit is configured to be worn by a human, and
the control unit causes the actuating unit to assist a motion of the human who drives a vehicle.

4. The control device according to claim 1, wherein
the information acquisition unit acquires at least one of an operation of a cook, and heat, a shape, a color, a texture, and an odor of a dish as information, and
the control unit controls the actuating unit by using the information and AI, and causes the actuating unit to reproduce a dish.

5. The control device according to claim 4, wherein
the actuating unit is configured to be worn by a human, and
the control unit causes the actuating unit to assist a motion of the human who cooks.

6. The control device according to claim 1, wherein
the information acquisition unit acquires an operation of surgery of a doctor, and
the control unit controls the actuating unit by using the information and AI, and causes the actuating unit to perform surgery.

7. The control device according to claim 6, wherein
the actuating unit is configured to be worn by a human, and
the control unit causes the actuating unit to assist a motion of the human who performs surgery.

8. The control device according to claim 1, wherein
the information acquisition unit acquires an operation of a factory worker, and
the control unit controls the actuating unit by using the information and AI, and causes the actuating unit to perform a factory work.

9. The control device according to claim 8, wherein
the actuating unit is configured to be worn by a human, and
the control unit causes the actuating unit to assist a motion of the human who performs the factory work.

10. A program for causing a computer to function as the control device according to any one of claims 1 to 9.

11. An operation reproducing device comprising:
an actuating unit that reproduces an operation of a model;
an information acquisition unit that acquires information related to the operation of the model from a sensor;
a designated item acquisition unit that acquires a designated item when reproducing the operation of the model; and
a control unit that causes the actuating unit to reproduce the operation based on the information acquired from the sensor and the designated item.

12. The operation reproducing device according to claim 11, wherein
the actuating unit is worn by a human, and
the control unit
causes the actuating unit to assist a human wearing the actuating unit to reproduce the operation.

13. The operation reproducing device according to claim 11, wherein
the information acquisition unit
acquires at least one of an operation of cooking by a cook who cooks, a time taken for each step of cooking, a shape and a size of an ingredient, and a temperature, a shape, a color, a texture, and an odor of a dish as information,
the designated item acquisition unit
acquires at least one of seasoning, a time by which the dish is desired to be completed, a type of the cook, and a level of the cook as the designated item, and
the control unit
causes the actuating unit to reproduce the operation of cooking based on the information acquired from the sensor and the designated item.

14. The operation reproducing device according to claim 11, wherein
the information acquisition unit
acquires at least one of a body motion, a line of sight, and a moving speed when an athlete plays as information,
the designated item acquisition unit
acquires a skill of a person who plays as the designated item, and
the control unit
causes the actuating unit to reproduce an operation of sports based on the information acquired from the sensor and the designated item.

15. The operation reproducing device according to claim 12, wherein
the actuating unit
feeds back information related to a motion of the human wearing the actuating unit to the designated item acquisition unit, and
the designated item acquisition unit
acquires the fed back information as a designated item.

16. An operation reproducing method executed by a computer, the computer functioning as:
an information acquisition unit that acquires information related to an operation of a model from a sensor; and
a designated item acquisition unit that acquires a designated item when reproducing the operation of the model, the method comprising
a step of causing an actuating unit that reproduces the operation of the model to reproduce the operation based on the information acquired from the sensor and the designated item.

17. An operation learning support device that supports a user to learn an operation of a model, the operation learning support device comprising:
an actuating unit that operates in accordance with given information;
a first information acquisition unit that acquires, from a first sensor, information related to the operation of the model;
a second information acquisition unit that acquires information related to an operation of the user via a second sensor worn by the user;
an extraction unit that compares the information related to the operation of the model with the information related to the operation of the user and extracts a difference between two; and
a control unit that generates, based on the difference between the two, complementary information for complementing difference in operation between the user and the model, and outputs the complementary information to the actuating unit.

18. The operation learning support device according to claim 17, further comprising:
a determination unit that determines an operation learning level of the user based on the difference between the two, and outputs a determination result, wherein
the control unit generates the complementary information for complementing the difference in operation between the user and the model based on the difference between the two and the determination result, and outputs the complementary information to the actuating unit.

19. The operation learning support device according to claim 17 or 18, further comprising a notification unit that notifies the user of the difference in operation when the difference between the two is extracted.

20. The operation learning support device according to claim 19, wherein
the control unit outputs the complementary information to the actuating unit and the notification unit, and
the notification unit notifies the difference in operation based on the complementary information.

21. The operation learning support device according to claim 20, wherein the operation of the model is an operation by a cook or an operation by an athlete.

22. A program for causing a computer that supports a user to learn an operation of a model to function as:
an actuating unit that operates in accordance with given information;
a first information acquisition unit that acquires, from a first sensor, information related to the operation of the model;
a second information acquisition unit that acquires information related to an operation of the user via a second sensor worn by the user;
an extraction unit that compares the information related to the operation of the model with the information related to the operation of the user and extracts a difference between two; and
a control unit that generates, based on the difference between the two, complementary information for complementing difference in operation between the user and the model, and outputs the complementary information to the actuating unit.
